# EUROPEAN PATENT APPLICATION

(11) **EP 2 151 234 A1**
(43) Date of publication of application: **10.02.2010**
(21) Application number: 08384010.8
(22) Date of filing: 28.07.2008
(51) Int. Cl.: A61K 9/16, A61K 9/20

(54) **Pharmaceutical formulation comprising a CB1-receptor compound in a solid solution and/or solid dispersion**

(71) Applicant: Laboratorios Del. Dr. Esteve, S.A., 08041 Barcelona (ES)
(72) Inventor: Soler Ranzani, Luis, 08013 BARCELONA (ES); Casadevall-Pujals, Gemma, 08019 BARCELONA (ES); Santanach-Delisau, Angel, 08500 VIC (Barcelona) (ES)
(74) Representative: Peters, Hajo

(57) **Abstract**

The present invention relates to a pharmaceutical formulation comprising 5-(4-chlorophenyl)-1-(2,4-dichlorophenyl)-N-(cis-2,6-dimethylpiperidin-1-yl)-4-methyl-4,5-dihydro-1H-pyrazole-3-carboxamide as racemate or (S)-enantiomer or mixtures thereof in a solid solution and/or solid dispersion.

## Description

The present invention relates to a pharmaceutical formulation comprising 5-(4-chlorophenyl)-1-(2,4-dichlorophenyl)-N-(2,6-dimethylpiperidin-1-yl)-4-methyl-4,5-dihydro-1H-pyrazole-3-carboxamide as racemate or (S)-enantiomer or mixtures thereof in a solid solution and/or solid dispersion.

Cannabinoids are compounds, which are derived from the cannabis sativa plant which is commonly known as marijuana. The most active chemical compound of the naturally occurring cannabinoids is tetrahydrocannabinol (THC), particularly Δ⁹-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated CB₁ and CB₂ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the CB₁-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Also dyslipidaemia, metabolic syndrome (both in its weight dependent or weight independent aspects) and diabetes type II have over the last decades risen in importance for the public health, especially for the developed or developing countries, likely due to a demoscopically marked increase in the proportion of obese or overweight members of the population or in average age.

The racemic compound denominated cis-5-(4-chiorophenyl)-1-(2,4-dichlorophenyl)-N-(2,6-dimethylpiperidin-1-yl)-4-methyl-4,5-dihydro-1H-pyrazole-3-carboxamide (the compound according to formula I below) is disclosed in WO07/09689 (see compound nr 103 on page 229); the content of this publication being included here by reference. For the synthesis of the compounds according to formulas (Ia) and (Ib) see below the experimental part. All of these compounds were shown to be CB1-binders and/or compounds with antiobesity activity and/or compounds having a reducing effect on triglyceride levels in blood in in-vivo models. Thus, these compounds according to formulas (I), (Ia) and (Ib) did show clear effects especially hinting at a great potential for the treatment or prevention of obesity, dyslipidaemia, metabolic syndrome (both in its weight dependent or weight independent aspects) and/or diabetes type II.

Thus, it was an object of the present invention to provide suitable pharmaceutical compositions for these compounds. As these compounds do have a tendency to be not highly soluble, this aspect - important when dealing with pharmaceutical compositions, especially those suitable to enhance the absorption of the active principle through a mucosa, especially for pharmaceutical compositions for oral administration - under certain therapeutical circumstances needs improvement.

Accordingly it would be desirable to find a formulation or a way to achieve a formulation in which the active principle would be realeased into an aqueous medium - simulating oral application - with a higher dissolution rate than is seen with the active principle alone. Especially it would be desirable to have a high dissolution rate in an aqueous medium like a 0.1 N hydrochloric acid - simulating the gastric acid - in the first 10 to 30 minutes like more than double of that seen in the active principle to be dissolved. Most preferably even much higher rates of dissolution in the first 10 to 30 minutes are desirable.

Also it would be desirable to find a formulation or a way to achieve a formulation in which the after oral administration in an in-vivo model the active principle would be found in blood plasma with a much higher concentration (measured as AUCₒ₋ₜ) and/or reaching Tₘₐₓ much faster compared to the active principle alone.

It would be even more desirable to find a formulation or a way to achieve a formulation
- in which the active principle would be realeased into an aqueous medium - simulating oral application - with a higher dissolution rate than is seen with the active principle alone
- and in which the after oral administration in an in-vivo model the active principle would be found in blood plasma with a much higher concentration (measured as AUCₒ₋ₜ) and/or reaching Tₘₐₓ much faster compared to the active principle alone.

Said object was achieved by providing a pharmaceutical composition comprising a solid dispersion and/or solid solution according to the invention and optionally further pharmaceutical acceptable ingredients.

Thus in another related aspect the invention further provides a solid dispersion and/or solid solution of one or more active principles selected from compounds of formula (I), (Ia) or (Ib) or mixtures thereof 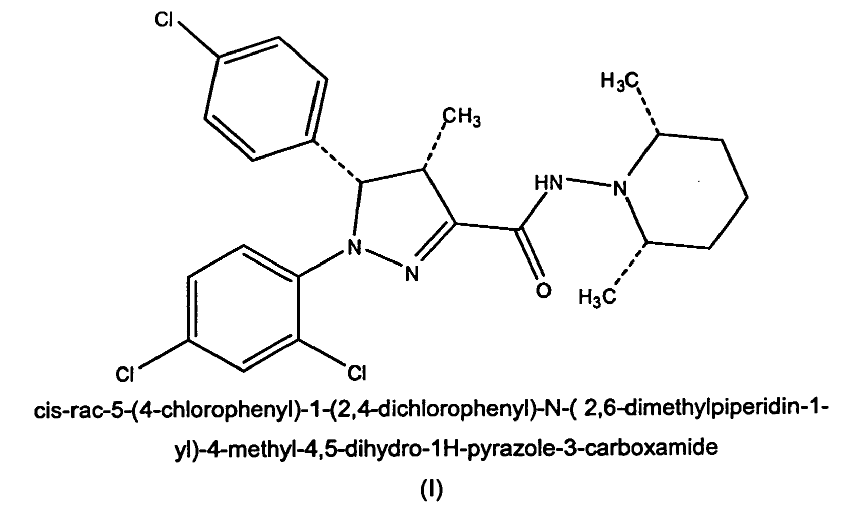 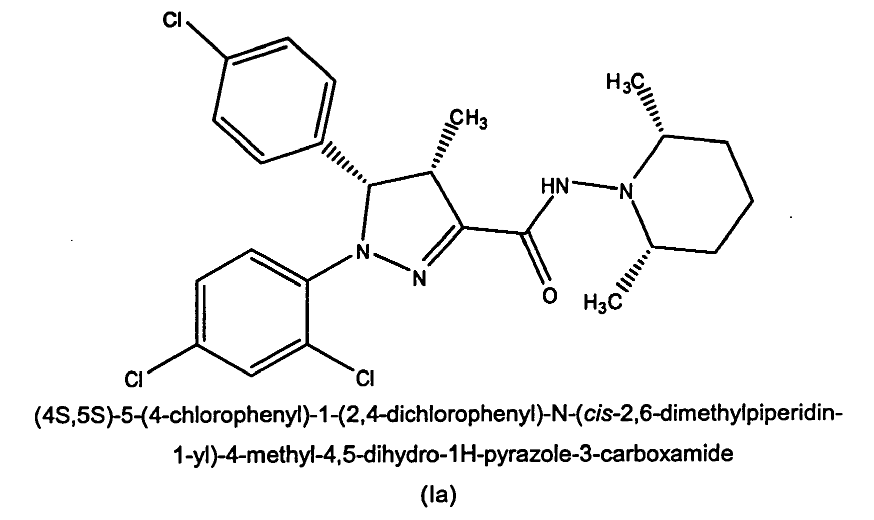 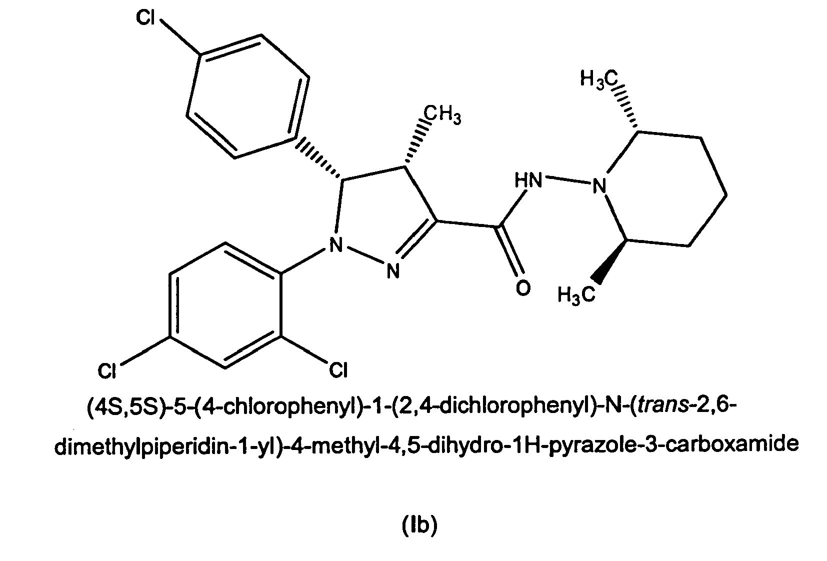 , optionally in form of one or more of the appropriate polymorphs, in amorphous form and/or corresponding salts and/or corresponding solvates thereof,
obtainable by a process in which
a) the active principle/s is/are mixed with an at least equimolar amount of at least one carrier,
b) thereafter or during step a) the mixture is heated until the carrier in the mixture is melted and
c) subsequently the - preferably eutectic - mixture is cooled below the melting point of the carrier in the mixture.

It is found that the release and uptake of the active principle may be greatly enhanced and improved by the pharmaceutical compositions according to the invention. Especially these compositions do show an improved dissolution or dissolution profile, mostly expressed as a higher dissolution rate of the active principle (e.g. expressed as mg/hr). As this is positively influencing also the uptake of the active principle into the body, as a consequence the pharmaceutical composition is allowing for a lowered effective dose to be used, which thus clearly also acts positively on any unwanted side effect. On the other hand the composition according to the invention by its improved dissolution rate results also in a higher ratio of the active principle, creating faster the - in some cases - necessary concentrations of the active principle in the peripherical system, while at the same time allowing thus for an even further reduction of the amount of active principle having to be applied to the patient. In addition the formulations according to the invention are also thus especially useful for the treatment of acute abuse e.g. as an emergency treatment after abuse of a medicament/drug.

"Solid dispersion and/or solid solution" is defined according to the invention as a dispersion or solution of the active principle in an inert carrier or matrix at solid state prepared through melting with a carrier or coprecipitation/coevaporation from a solution with a carrier and a solvent. Preferably the active principle/s is/are finely dispersed/dissolved up to and including single molecules being dispersed/dissolved in the carrier. During the preparation by melting a physical mixture of at least one active principle is mixed with at least one carrier, melted (at least the carrier in the mixture) and the melt rapidly solidified by lowering the temperature. During the preparation by coprecipitation/coevaporation from a solution carrier and active principle are dissolved in a solvent, mixed and the solvent removed (usually evaporated or maybe freezedried or spraydried). Most preferably according to the invention "solid dispersion and/or solid solution" is defined as a dispersion of the active principle in an inert carrier or matrix at solid state prepared through melting.

"Carrier" in the sense of this application is a substance in which one or more active principles may be dispersed/dissolved, preferably finely dispersed/dissolved up to and including single molecules being dispersed in the carrier. Preferably a carrier is defined by one or more, preferably most, more preferably all - where applicable - of the following criteria:
- be freely water-soluble with intrinsic rapid dissolution properties;
- be nontoxic and pharmacologically inert;
- be heat stable with low/med melting point (if melting is used for the preparation of the solid dispersion);
- be soluble in a variety of solvents and pass through a vitreous state upon solvent evaporation (if solvent evaporation is used for the preparation of the solid dispersion);
- be able to increase the aqueous solubility of at least on of the active principles;
- be chemically compatible with the drug and not form a strongly bonded complex with any of the active principles dispersed therein.
   Further examples of carriers may be found in Ford, J.L.; Acta Helv. 1986, 61, 69-88, enclosed here by reference.

"Hot melt extrusion" is defined as an extrusion of the active principle/s together with the carrier/s in an extruder, where the carrier/s and active principle/s are mixed and melted or softened by melting the carrier before or during extrusion thus creating upon extrusion a solid dispersion and/or solid solution (see above). Hot-melt extrusion is described e.g. by Koleng at al. "Hot-Melt Extrusion Technology", Encyclopedia of Pharmaceutical Technology (2002), Macel Dekker, Inc.

The active principle according to the invention may be subject to any particle size reduction like milling, grinding, nanosizing or micronisations, or to complexation like e.g. complexation with Ciclodextrines, or PEG.

In a preferred embodiment of the solid dispersion and/or solid solution according to the invention the carrier is either hydrophilic or hygroscopic and has a melting point between +40°C and the melting point of the active principle or mixture of active principles +20 °C.

Preferably "hydrophilic" means in the context of this application that the carrier shows an HLB (hydrophilic-lipophilic balance) determined according to the method of W. C. Griffin (1954) of 12 to 20, more preferably 15 to 20.

In another preferred embodiment of the solid dispersion and/or solid solution according to the invention the carrier is selected from
- sugars, like dextrose, sucrose, galactose, sorbitol, maltose, xylitol, manitol, lactose;
- acids, like citric acid, succinic acid, ascorbic acid;
- polymeric materials, like povidone (PVP), polyethylene oxide (PEO), polyethylene glycol (PEG), hydroxpropylmethylcellulose (HPMC), methylcellulose (MC), ethylcellulose (EC), hydroxyethylcelllose (HEC), cylodextrin, hydroxypropylcellulose (HPC), pectin, galactomannan;
- insoluble or enteric polymers, like hydroxypropylmethylcellulose phthalate, polymethacrylates (e.g. Eudragit L-100, Eudragit S-100, Eudragit RL, Eudragit RS, Eudragit EPO);
- surfactants, like polyoxyethylene stearate, renex, poloxamer 188, texafor, AIP, deoxycholic acid, polyoxy-ethylene-sorbitan higher fatty acid esters (e.g. Tween, like Tween 80), spans;
- others, consisting of: camuba wax, pentaerythritol, pentaerythrityltetraacetate, urea, urethane, hydroxyalkylxanthins;
   preferably the carriers are selected from
   EUDRAGIT, MYRJ 52, VITE-TPGS, GELUCIRE 50/13, HPMC-PHTALATE, HPMC, HEC, HPC-SL, PEO and/or POLOXAMER;

In a very preferred embodiment of the solid dispersion and/or solid solution according to the invention the carrier is an Eudragit, like Eudragit EPO, Eudragit L-100, Eudragit S-100, Eudragit RL, or Eudragit RS.

In another preferred embodiment of the solid dispersion and/or solid solution according to the invention
- the melting temperature in step (b) is in the range between +40°C and the melting point of the active principle or mixture of active principles +20 °C; and/or
- the cooling step (c) is done by lowering the temperature by more than 10°C/sec, preferably 20°C/sec, down to temperatures below 25 °C, preferably below 0 °C; and/or is done by contacting the melt of step (b) with an environment having a temperature of 25°C or lower, preferably 0°C or lower.

In another preferred embodiment of the solid dispersion and/or solid solution according to the invention the molecular ratio between carrier and active principle is between 1:1 and 1: 20, preferably is between 1:1 and 1:10, more preferably is between 1:2 and 1:5.

In another aspect the invention is referring to a pharmaceutical composition comprising the solid solution and/or solid dispersion according to the invention and optionally further pharmaceutical acceptable ingredients.

In a preferred embodiment of the pharmaceutical composition according to the invention
- the active principle is present in an amount of 10 to 60, preferably 20 to 40 % by weight based on the total weight of the composition;
   and/or
- the active principle is present in an amount of 1 to 250 mg, preferably 10 to 200 mg, more preferably 15 to 150 mg in the composition.

In another preferred embodiment of the pharmaceutical composition according to the invention it is suitable to enhance the absorption of the active principle through a mucosa.
Thereby it is preferred if the mucosa is selected from the
- nasal or olfactory mucosa,
- buccal or oral mucosa,
- gastric mucosa,
- intestinal mucosa
- vaginal mucosa, or
- rectal mucosa,
   preferably selected from
- gastric mucosa, or
- intestinal mucosa.

In another preferred embodiment of the pharmaceutical composition according to the invention the pharmaceutical composition is selected from
- a pharmaceutical composition for oral application,
- a pharmaceutical composition for nasal application,
- a pharmaceutical composition for buccal application,
- a pharmaceutical composition for rectal application, or
- a pharmaceutical composition for vaginal application;
   or
- a pharmaceutical composition for transdermal application;
- a pharmaceutical composition for systemic application;
   preferably selected from
- a pharmaceutical composition for oral application.

In another preferred embodiment of the pharmaceutical composition according to the invention the pharmaceutical composition is selected from
- a tablet,
- a capsule,
- a sachet,
- a powder,
- a caplet,
- a gel,
- a film,
- a pellet,
- a granule,
- an implant,
- a multiparticulate with granules compressed into a tablet,
- a multiparticulate with granules filled into a capsule,
- a multiparticulate with pelets compressed into a tablet,
- a multiparticulate with pelets filled into a capsule, or
- a suppository,
   or
- an injectable solution/dispersion
- a transdermal system like a patch;
   preferably selected from
- a tablet,
- a capsule,
- a pellet,
- a granule,
- a multiparticulate with granules compressed into a tablet,
- a multiparticulate with granules filled into a capsule,
- a multiparticulate with pelets compressed into a tablet, or
- a multiparticulate with pelets filled into a capsule.

In another preferred embodiment of the pharmaceutical composition according to the invention
- the further pharmaceutical acceptable ingredients are present in a total amount of 5 to 95 %, preferably 50 to 90 % by weight based on the total weight of the composition;
   and/or
- the further pharmaceutical acceptable ingredients are present in a total amount of 0 to 300 mg, preferably 0 to 250 mg, more preferably 20 to 250 mg.

In another preferred embodiment of the pharmaceutical composition according to the invention the further pharmaceutical acceptable ingredient/s is/are selected from a diluent, a binder and/or a lubricant, and/or optionally a flowing agent, an antiadhesive, a preservative, and/or, optionally, a taste masking agent, a coloring agent and/or a flavoring agent and/or a permeation enhancer.

In another preferred embodiment of the pharmaceutical composition according to the invention the pharmaceutical composition is prepared using hot-melt extrusion.

Another preferred aspect of the invention is a pharmaceutical composition comprising as active principle at least one of
- (cis-rac)- 5-(4-chlorophenyl)-1-(2,4-dichlorophenyl)-N-(2,6-dimethylpiperidin-1-yl)-4-methyl-4,5-dihydro-1H-pyrazole-3-carboxamide;
- (4S,5S)-5-(4-chlorophenyl)-1-(2,4-dich)orophenyl)-N-(*cis*-2,6-dimethylpiperidin-1-yl)-4-methyl-4,5-dihydro-1H-pyrazole-3-carboxamide , or
- (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide ,
- or mixtures thereof;
   optionally in form of one or more of the appropriate polymorphs, in amorphous form and/or corresponding salts and/or corresponding solvates thereof,
   in a solid dispersion and/or solid solution in a carrier, the carrier having a melting point between +40°C and the melting point of the active principle +20 °C and being hydrophilic or hygroscopic;
   and optionally further pharmaceutical acceptable ingredients

Preferred concentration/weight/percentage ranges, forms of the compositions, as well as appropriate lists of preferred excipients or carriers have already been described above.

In a preferred embodiment of any of the pharmaceutical compositions or the solid dispersion according to the invention described above the active principle is selected from
a) (cis-rac)-5-(4-chlorophenyl)-1-(2,4-dichlorophenyl)-N-(2,6-dimethylpiperidin-1-yl)-4-methyl-4,5-dihydro-1H-pyrazole-3-carboxamide;
   preferably in
   - crystalline form;
   - amorphous form;
   - form of a solvate, preferably a hydrate, more preferably a monohydrate;
   - the free base; or
   - a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
b) (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, preferably
   preferably in
   - crystalline form;
   - amorphous form;
   - form of a solvate, preferably a hydrate,
   - the free base;
   - a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
c) (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide,
   preferably in
   - crystalline form;
   - amorphous form;
   - form of a solvate, preferably a hydrate,
   - the free base;
   - a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1 ,5-disulfonic acid, nitric acid, phosophoric acid, *p*-toluenesulfonic acid, sulfuric acid and/or thiocyanic acid; more preferably the salt being a hydrochloride;
      or
d) a nonracemic mixtures of (b) and (c).

The crystalline form of (cis-rac)- 5-(4-chlorophenyl)-1-(2,4-dichlorophenyl)-N-(2,6-dimethylpiperidin-1-yl)-4-methyl-4,5-dihydro-1H-pyrazole-3-carboxamide; especially (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide and (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide is described below. The amorphous form of (cis-rac)- 5-(4-chlorophenyl)-1-(2,4-dichlorophenyl)-N-(2,6-dimethylpiperidin-1-yl)-4-methyl-4,5-dihydro-1H-pyrazole-3-carboxamide; especially (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide and (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide is described below.

Solid solutions and dispersions of salts with an acid with a pkₐ ≤ 3.0 of (cis-rac)- 5-(4-chlorophenyl)-1-(2,4-dichlorophenyl)-N-(2,6-dimethylpiperidin-1-yl)-4-methyl-4,5-dihydro-1H-pyrazole-3-carboxamide; especially (4S,5S)-5-(4-chlorophenyl)-1-(2,4-dichlorophenyl)-N-(*cis-*2,6-dimethylpiperidin-1-yl)-4-methyl-4,5-dihydro-1H-pyrazole-3-carboxamide and (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide are also included in the scope of this invention. Specifically included are salts of the the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1 ,5-disulfonic acid, nitric acid, phosophoric acid, *p*-toluenesulfonic acid, sulfuric acid and/or thiocyanic acid. Some salts like e.g. hydrochloride salts etc. of (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide have also been described below.

The daily dosage for humans and animals may vary depending on factors that have their basis in the respective species or other factors, such as age, sex, weight or degree of illness and so forth. The daily dosage for humans may preferably be in the range from 10 to 400, preferably 15 to 300, more preferably 25 to 250 milligrams of active substance to be administered during one or several intakes per day. Most preferably is a dosage of 25 to 250 mg of active principle in one dosage unit.

The pharmaceutical acceptable ingredients excipients which optionally may be included in the composition according to the present invention include especially a diluent, a binder and/or a lubricant, wheras a flowing agent, a preservative, an antiadhesive and, optionally, a taste masking agent and a coloring agent and/or a flavoring agent as well as one or more permeation enhancers can also be added.

The diluent - if used - in the composition of the present invention can be one or more compounds which are capable of densifying the active principle to give the desired mass. The preferred diluents are inorganic phosphates such as calcium phosphates; sugars such as hydrated or anhydrous lactose, or mannitol; and cellulose or cellulose derivatives such as, for example, microcrystalline cellulose, starch, corn starch or pregelatinized starch. Lactose monohydrate, mannitol, microcrystalline cellulose and corn starch, used by themselves or in a mixture, for example a mixture of lactose monohydrate and corn starch, are very particularly preferred. The diluent - if present at all - is present in a proportion of 5 % to 90 % by weight based on the total weight of the composition according to the invention

The binder - if employed - in the composition of the present invention can be one or more compounds which are capable of densifying the active principle within the overall e.g. granular formulation by converting it to larger and denser particles with improved solid characteristics. The preferred binders are alginic acid or sodium alginate; cellulose and cellulose derivatives such as sodium carboxymethyl cellulose, ethyl cellulose, hydroxyethyl cellulose, hydroxypropyl cellulose, hydroxypropyl methyl cellulose or methyl cellulose; gelatin; acrylic acid polymers; and povidone, for example povidone K 30, which is a very particularly preferred binder. The binder - if present at all - is present in a proportion of 1% to 30% by weight based on the total weight of the composition according to the invention.

The lubricant - if employed - in the composition of the present invention can be one or more compounds which are capable of preventing the problems associated with the preparation of the dosage form, such as the sticking and/or seizing problems which occur in the machines during compression or filling. The preferred lubricants are fatty acids or fatty acid derivatives such as calcium stearate, glyceryl monostearate, glyceryl palmitostearate, magnesium stearate, sodium laurylsulfate, sodium stearylfumarate, zinc stearate or stearic acid; hydrogenated vegetable oils, for example hydrogenated castor oil; polyalkylene glycols, especially polyethylene glycol; sodium benzoate; or talcum. Magnesium stearate is preferred according to the present invention. The lubricant - if present at all - is present in a proportion of 0.2% to 5% by weight based on the total weight of the composition according to the invention.

The antiadhesive which may be employed in the composition of the present invention can be one or more compounds which are capable of reducing the sticky character of the formulation, for example of preventing adhesion to metal surfaces. The preferred antiadhesives are compounds containing silicon, for example silica or talcum. The antiadhesive - if present at all - can be present in a proportion of 0 to 5% by weight in the pharmaceutical composition according to the invention.

The flowing agent which may be employed in the composition of the present invention can be one or more compounds which are capable of facilitating the flow of the prepared formulation. The preferred flowing agents are compounds containing silicon, for example anhydrous colloidal silica or precipitated silica. The flowing agent - if present at all - can be present in a proportion of 0 to 15% by weight in the pharmaceutical composition according to the invention.

The permeation enhancer which may be employed in the composition of the present invention, especially if the composition is a transdermal formulation, can be one or more compounds which are capable of facilitating the permeation of the active principle from the composition trough a biological barrier like e.g. the skin. Preferred permeation enhancers are high-boiling alcohols, diols, fatty acid esters, oleic acid and glyceride-based solvents, like for example isobutyl or isopropyl alcohol. Other permeation enhancers include linoleic acid, oleic acid, dimethylacetamide, lauric acid, myristic acid, palmitic acid, dimethyl sulfoxide, glycofurol, thymol, pyrrolidone, macrogol 15 hydroxystearate; mineral oil, light; polyoxyethylene alkyl ethers, menthol, polycarbophil, isopropyl myristate, glyceryl monooleate, ethyl acetate, polyethylene glycol, carbomer. The permeation enhancer/s - if present at all - can be present in a proportion of 0 to 25% by weight in the pharmaceutical composition according to the invention.

According to the present invention, the pharmaceutical compositions may preferably be prepared by a direct compression process, a granulation, extrusion and further spheronization, or by a layering process into for example nonpareil seeds.

Thus, for the direct compression process the active principle in a solid solution and/or solid dispersion and the optional diluent, binder are combined by geometrical mixing and further mixed with other optional excipients such colorants or taste masking agents, afterwards the mixture can be either compressed, encapsulated or dosified into sachets or any other unidose system.

For the dry granulation process the internal phase, thus the active principle in a solid solution and/or solid dispersion, the optional diluent, the optional binder, and, optionally, the coloring agent are mixed optionally at room temperature. The ingredient or ingredients of the optional external phase, namely the optional lubricant, possibly the antiadhesive, the flowing agent and, if appropriate, the coloring agent and/or the flavoring agent, are then added to the graded dry grains.

For the extrusion-espheronization process, a mass comprising the active principle in a solid solution and/or solid dispersion - for example the one obtained in the previous pharagraph - is passed through a pharmaceutical extruder and further spheronized until obtaining multiparticulates containing the active principle of a desired particle size. Later on these particles are dried and susceptible to either encapsulation or added into sachets (adding lubricants and other required processing agents), compressed (if mixed with the proper compression base) or coated for further manipulation, e.g. further layering (see below).

In one embodiment of the layering process to an inert sugar/starch/cellulose spherical core, a layer is applied containing a mixture of the active principle in a solid solution and/or solid dispersion, the optional diluent, the optional binder, and, optionally, the coloring agent, optionally followed by a another - e.g. isolation - layer formed by water soluble polymers and compatible excipients. Finally, optionally a layer consisting of a controlled release coating - like e.g. an enteric coating - is applied.

In another possibility of the layering process to a spherical core comprising already the active principle in a solid solution and/or solid dispersion produced by e.g. the extrusion-spheronization process described above, optionally one or more futher layers are applied containing a mixture of e.g. the optional diluent, the optional binder, and, optionally, the coloring agent, optionally water soluble polymers and compatible excipients. Finally, optionally a layer consisting of a controlled release coating - like e.g. an enteric coating - is applied.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

In another preferable aspect of the invention the pharmaceutical composition according to the invention is suitable for the modulation (regulation) of cannabinoid-receptors, preferably cannabinoid 1 (CB₁) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferably said pharmaceutical composition is suitable for the prophylaxis and/or treatment of psychosis and also depression as well as memory disorders.

Also particularly preferably said pharmaceutical composition is suitable for the prophylaxis and/or treatment of neuropathic pain, hyperalgesia or allodynia.

Also particularly preferably said pharmaceutical composition is suitable for the prophylaxis and/or treatment of food intake disorders, preferably bulimia, anorexia, cachexia, obesity and/or type II diabetus mellitus (non-insuline dependent diabetes mellitus), more preferably obesity and/or type II diabetus mellitus (non-insuline dependent diabetes mellitus). The inventive medicament also seems to be active in the prophylaxis and/or treatment of appetency disorders, e.g. the pyrazoline compounds of general formula I also reduce the desire for sweets and other macronutrients. In addition said pharmaceutical composition is also suitable for the prophylaxis and/or treatment of metabolic syndrome (both in its weight dependent or weight independent aspects) and dyslipidaemia.

Also particularly preferably said pharmaceutical composition is suitable for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said pharmaceutical composition is suitable for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments and/or drugs, which are frequently the subject of misuse include opioids, barbiturates, cannabis, cocaine, amphetamines, phencyclidine, hallucinogens and benzodiazepines. The formulations according to the invention are especially useful for the treatment of acute abuse e.g. as an emergency treatment after abuse of any of the medicaments/drugs mentioned above.

The pharmaceutical composition according to the invention is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

In an embodiment of the pharmaceutical composition according to the invention the medicament is for the regulation of triglyceride levels in the blood plasma and for the prophylaxis and/or treatment of disorders of the central nervous system, especially stroke, of disorders of the cardiovascular system and of of food intake disorders, preferably bulimia, anorexia, cachexia, obesity, type II diabetus mellitus (non-insuline dependent diabetes mellitus), preferably obesity and diabetis.

In an embodiment of the pharmaceutical composition according to the invention the composition is for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

In an embodiment of the pharmaceutical composition according to the invention the composition is for the modulation of cannabinoid-receptors, preferably cannabinoid 1 (CB₁) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

The following part of the description is exemplifying the invention and is not meant to limit it.

### Examples:

### Example 1: Blend 1 (solid solution)

10 weight % of the final solid solution of active principle (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide is mixed with 90 weight % of the final solid solution of Eudragit EPO in an apparatus suitable for hot-Melt Extrusion. The mixture is extruded with an extrusion temperature of 150°C and instantly cooled. The solid solution can then be formulated with excipient by granulation into pellets or compressed into tablets.

### Example 2: Blend 2 (solid solution)

30 weight % of the final solid solution of active principle (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide is mixed with 70 weight % of the final solid solution of Eudragit EPO in an apparatus suitable for hot-Melt Extrusion. The mixture is extruded with an extrusion temperature of 187°C and instantly cooled. The solid solution can then be formulated with excipient by granulation into pellets or compressed into tablets.

### Example 3: Blend 2 (solid dispersion)

30 weight % of the final solid dispersion of active principle (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide is mixed with 70 weight % of the final solid dispersion of Eudragit EPO in an apparatus suitable for hot-Melt Extrusion. The mixture is extruded with an extrusion temperature of 150°C and instantly cooled. The solid dispersion can then be formulated with excipient by granulation into pellets or compressed into tablets.

### Example 4: Blend 3 (solid solution)

50 weight % of the final solid solution of active principle (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide is mixed with 50 weight % of the final solid solution of Eudragit EPO in an apparatus suitable for hot-Melt Extrusion. The mixture is extruded with an extrusion temperature of 187°C and instantly cooled. The solid solution can then be formulated with excipient by granulation into pellets or compressed into tablets.

### Example 5: Blend 3 (solid dispersion)

50 weight % of the final solid dispersion of active principle (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide is mixed with 50 weight % of the final solid dispersion of Eudragit EPO in an apparatus suitable for hot-Melt Extrusion. The mixture is extruded with an extrusion temperature of 150°C and instantly cooled. The solid dispersion can then be formulated with excipient by granulation into pellets or compressed into tablets.

### Example 6: Overview of Examples 1 to 5

Formulations: 3 different blends of the active principle, ((4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, and Eudragit EPO are formed as shown in Table I.

**Table I. Formulation composition**

| Component | Blend 1 | Blend 2 | Blend 3 |
|---|---|---|---|
| Active Principle | 10% | 30% | 50% |
| Eudragit EPO | 90% | 70% | 50% |

Hot Melt Extrusion: Haake Minilab equipped with two co-rotatory screws is used to obtain extrudate strands. Extrusion temperature applied to the three blends is shown in Table II. Obtention of solid solutions or solid dispersions depends on extrusion temperarure.

**Table II. Extrusion temperature and solid state of extrudate strands.**

| Temperature | Blend 1 | Blend 2 | Blend 3 |
|---|---|---|---|
| 150°C | Solid solution (Ex. 1) | Solid dispersion (Ex. 3) | Solid dispersion (Ex. 5) |
| 187°C | --- | Solid solution (Ex. 2) | Solid solution (Ex. 4) |

### Example 7: (solid solution)

90 mg of the active principle (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide is mixed with 210 mg Eudragit EPO. The mixture is melted and after melting instantly cooled in an ice bath.
The solid solution/dispersion can then be formulated with excipient by granulation into pellets or compressed into tablets.

| **FINAL COMPOSITION** | **mg** | **%** |
|---|---|---|
| (4S,5S)-5-(4-chlorophenyl)-1-(2,4-dichlorophenyl)-N-(*cis*-2,6-dimethylpiperidin-1-yl)-4-methyl-4,5-dihydro-1 H-pyrazole-3-carboxamide (Pellets) | 90 | 30 |
| Eudragit EPO | 210 | 70 |

### Example 8: (hot melt extrusion)

90 mg of the active principle (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide is mixed with 180 mg Eudragit EPO and 30 mg of Vitamin E and subsequently melted and extruded in a holt-melt extruder. The solid solution/dispersion can then be formulated with excipient by granulation into pellets or compressed into tablets.

| **FINAL COMPOSITION** | **mg** | **%** |
|---|---|---|
| (4S,5S)-5-(4-chlorophenyl)-1-(2,4-dichlorophenyl)-N-(*cis*-2,6-dimethylpiperidin-1-yl)-4-methyl-4,5-dihydro-1 H-pyrazole-3-carboxamide (Pellets) | 90 | 30 |
| Eudragit EPO | 180 | 60 |
| Vitamin E | 30 | 10 |

### Example 9 (Comparative example-Tablet):

1) Granulation step: In a wet low-shear granulator the following excipients are mixed :
   - The active principle (4S,5S)-5-(4-chlorophenyl)-1-(2,4-dichlorophenyl)-N-(*cis-*2,6-dimethylpiperidin-1-yl)-4-methyl-4,5-dihydro-1H-pyrazole-3-carboxamide,
   - Lactose
   - Maize starch-part
      The blend is mixed for 2 minutes and then, while under chopper and mixer action a solution containing PVP K 30 is incorporated. The mixture is granulated and the granulator unloaded.
2) Sieving (1): The final product is sieved to avoid agglomerates.
3) Drying: The final product from step 3 is dried using a fluid bed at 50°C until relative humidity is below 2%.
4) Blending: External phase excipients are added to the dry granule (Maize starch-part; SLS, CMC and Mg Stearate)
5) Sieving (2): The final product is sieved to avoid agglomerates.
6) Compression step:

| **FINAL COMPOSITION** | **%** |
|---|---|
| (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide | 28,4 |
| Maize Starch | 22,7 |
| Lactose Monohydrate | 42,6 |
| PVP K30 | 2,8 |
| SLS | 0,1 |
| CMC | 2,6 |
| Mg Stearate | 0,9 |

Compressed as tablets.

### Example 10 (Comparative example-Non-excipients):

90 mg of the active principle (4S,5S)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide was placed into #1 hard gelatin capsules.

### Example 11: (solid solution, trans-2,6-enantiomer)

90 mg of the active principle (4S,5S)-5-(4-chlorophenyl)-l-(2,4-dichlorophenyl)-N-(*trans*-2,6-dimethylpiperidin-1-yl)-4-methyl-4,5-dihydro-1H-pyrazole-3-carboxamide is mixed with 210 mg Eudragit EPO. The mixture is melted and after melting instantly cooled in an ice bath. The solid solution/dispersion can then be formulated with excipient by granulation into pellets or compressed into tablets.

| **FINAL COMPOSITION** | **mg** | **%** |
|---|---|---|
| (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide (Pellets) | 90 | 30 |
| Eudragit EPO | 210 | 70 |

### Example 12: (hot melt extrusion, trans 2,6-enantiomer)

90 mg of the active principle (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide is mixed with 180 mg Eudragit EPO and 30 mg of Vitamin E and subsequently melted and extruded in a holt-melt extruder. The solid solution/dispersion can then be formulated with excipient by granulation into pellets or compressed into tablets.

| **FINAL COMPOSITION** | **mg** | **%** |
|---|---|---|
| (4S,5S)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide (Pellets) | 90 | 30 |
| Eudragit EPO | 180 | 60 |
| Vitamin E | 30 | 10 |

### Example 13: (solid solution, racemic)

90 mg of the active principle (rac)- 5-(4-chlorophenyl)-1-(2,4-dichlorophenyl)-N-(2,6-dimethylpiperidin-1-yl)-4-methyl-4,5-dihydro-1H-pyrazole-3-carboxamide is mixed with 210 mg Eudragit EPO. The mixture is melted and after melting instantly cooled in an ice bath. The solid solution/dispersion can then be formulated with excipient by granulation into pellets or compressed into tablets.

| **FINAL COMPOSITION** | **mg** | **%** |
|---|---|---|
| (rac)-5-(4-chlorophenyl)-1-(2,4-dichlorophenyl)-N -(2,6-dimethylpiperidin-1-yl)-4-methyl-4,5-dihydro-1H-pyrazole-3-carboxamide (Pellets) | 90 | 30 |
| Eudragit EPO | 210 | 70 |

### Example 14: (hot melt extrusion, racemic)

90 mg of the active principle (rac)- 5-(4-chlorophenyl)-1-(2,4-dichlorophenyl)-N-(2,6-dimethylpiperidin-1-yl)-4-methyl-4,5-dihydro-1H-pyrazole-3-carboxamide is mixed with 180 mg Eudragit EPO and 30 mg of Vitamin E and subsequently melted and extruded in a holt-melt extruder. The solid solution/dispersion can then be formulated with excipient by granulation into pellets or compressed into tablets.

| **FINAL COMPOSITION** | **mg** | **%** |
|---|---|---|
| (rac)- 5-(4-chlorophenyl)-1-(2,4-dichlorophenyl)-N-(2,6-dimethylpiperidin-1-yl)-4-methyl-4,5-dihydro-1H-pyrazole-3-carboxamide (Pellets) | 90 | 30 |
| Eudragit EPO | 180 | 60 |
| Vitamin E | 30 | 10 |

### PHARMACOLOGICAL EXAMPLES:

### IN-VITRO TESTS:

### P1: In-vitro dissolution:

Dissolution profiles of the solid solutions/solid dispersions according to Examples 1 to 5 are performed with USP Apparattus 2, paddles at 100 rpm and 1000 ml of HCl 0.1 N as dissolution media. Vessel temperature is 37°C.

### IN-VIVO TESTS:

### P2: In-vivo dissolution:

A pharmacokinetic study in rats is performed with the formulations according to Examples 1 to 5 compared to the active principle alone. The formulation according to Examples 1 to 5 as well as the active principle alone are given orally at a concentration 10 mg/kg (of active principle) to male Wistar Hannover rats. Plasma samples are centrifugated and after protein precipitation are analyzed by HPLC.

### CHEMICAL EXAMPLES:

### Chemical -Example 1: Preparation of (E)-4-(4-Chlorophenyl)-3-methyl-2-oxo-but-3-enoic acid

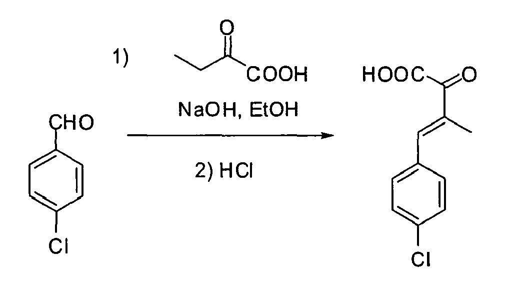

To a solution of aqueous 0.5 M NaOH (85.2 g, 2.13 mol 1.5 eq) in water (4.26 L), under N₂ at room temperature, 2-oxobutyric acid (159.7 g, 1.56 mol, 1.1 eq) was added in portions (60 mL of EtOH were used to wash the product container). The reaction was then left stirring for 5 min and a solution of 4-chlorobenzaldehyde (200.0 g, 1.42 mol, 1 eq) in abs. EtOH (710 mL) was then slowly added (approx. rate of addition: 2 h at 150 mL/h and 7h at 50 mL /h). The reaction was left to stir at 25 °C overnight. Water was added (800 mL) and the solution evaporated under reduced pressure to eliminate the excess of EtOH. The solution was then washed with toluene and evaporated (3 x 500 mL) to eliminate traces of this solvent. The aqueous solution was then cooled down in an ice bath and conc. HCl (240 mL) was slowly added under magnetically stirring. A white solid precipitated from the solution which was kept at 0 °C for another hour. The solid was filtered under vacuum through a sintered funnel (porosity 3) and dried at 40 °C under vacuum (287.8 g, 86% yield).
¹H NMR (400 MHz, CDCl₃): δ 2.17 (3H, s, CH₃), 7.44 (4H, ap d, *J* 3.28 Hz, ArH), 8.41 (1H, s, CH); ¹³C NMR (100 MHz, CDCl₃): δ 13.2 (CH₃), 129.2 (CH), 129.6 (C), 131.9 (CH), 133.4 (C), 136.4 (C), 147.6 (CH), 164.4 (CO), 187.1 (CO).

### Chemical-Example 2: Preparation of racemate cis-5-(4-chlorophenyl)-1-(2,4-dichlorophenyl)-4-methyl-4,5-dihydro-1H-pyrazole-3-carboxylic acid

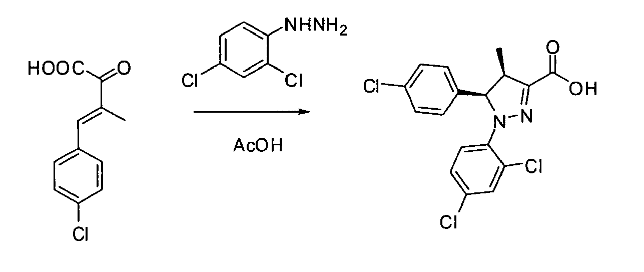

To a suspension of 2,4-dichlorophenylhydrazine hydrochloride (277.5 g, 1.27 mol, 1 eq) in acetic acid (2.0 L) at 80 °C, a solution of crude (E)-4-(4-chlorophenyl-3-methyl-2-oxo-3-butenoic acid (286.2 g, 1.27 mol) in glacial acetic acid (1.27 L) was slowly added and the reaction was maintained at 80 °C for 2 h. The reaction mixture was then allowed to cool down to 50 °C and concentrated under reduced pressure to approximately 2/3 of its initial volume. The solution was mechanically stirred at room temperature overnight and a yellow precipitated was formed. The solid was then filtered under vacuum through a sintered funnel (porosity 3) to obtain a mixture of racemates *cis* and *trans* 5-(4-chlorophenyl)-1-(2,4-dichlorophenyl)-4-methyl-4,5-dihydro-1*H*-pyrazol-3-carboxylic acid (430 g, 88.4 % yield) as a yellow solid, which was suspended in water (1.0 L), stirred for 30 min and filtered. This operation was repeated four times (until the pH of the filtered water was between 5 and 6). The solid was then left to dry under vacuum at 40 °C for 48 h (249 g, 51.2 % yield). The solid was suspended in toluene (1125 mL) and heated to 80 °C. The suspension was transformed into a solution, which was left at this temperature for 10 min and then at room temperature overnight. The following morning a yellow precipitated had formed. Filtration through a Buchner funnel under vacuum provided a whitish powdery solid of pure racemate *cis*-5-(4-chlorophenyl)-1-(2,4-dichlorophenyl)-4-methyl-4,5-dihydro-1*H*-pyrazol-3-carboxylic acid (189.7 g, 39 % yield, 76 % yield of crystallization).

IR (NaCl film, cm⁻¹) of the racemate *cis*-5-(4-chlorophenyl)-1-(2,4-dichlorophenyl)-4-methyl-4,5-dihydro-1*H*-pyrazol-3-carboxylic acid, measured with FT-IR Nexus by Thermo Nicolet: 2976, 1682, 1566, 1542, 1490, 1270, 1242, 1117.

¹H NMR (400 MHz, CHLOROFORM-*d*) δ ppm 0.96 (d, *J*=7.42 Hz, 3 H) 3.82 (td, *J*=11.72, 7.42 Hz, 1 H) 5.91 (d, *J*=11.72 Hz, 1 H) 7.04 (d, *J*=8.60 Hz, 2 H) 7.11 (dd, *J*=8.60, 2.34 Hz, 1 H) 7.21 - 7.30 (m, 4 H)

¹³C NMR (100 MHz, CDCl₃): δ 13.6 (CH₃), 43.5 (CH), 72.0 (CH), 124.9 (CH), 127.6 (CH), 129.1 (CH), 129.6 (CH), 130.9 (CH), 131.3 (C), 132.7 (C), 134.5 (C), 138.7 (C), 144.6 (C), 165.9 (CO).

### Chemical-Example 3: Separation of (4R, 5R) and (4S, 5S)-5-(4-chlorophenyl)-1-(2,4-dichlorophenyl)-4-methyl-4,5-dihydro-1H-pyrazole-3-carboxylic acid from racemate cis-5-(4-chlorophenyl)-1-(2,4-dichlorophenyl)-4-methyl-4,5-dihydro-1H-pyrazole-3-carboxylic acid

a) Separation by chiral HPLC.
   Conditions for the isolation of each enantiomer of racemate cis-5-(4-chlorophenyl)-1-(2,4-dichlorophenyl)-4-methyl-4,5-dihydro-1H-pyrazole-3-carboxylic acid by preparative chiral HPLC were the following:
   - Column: 250 x 20 mm CHIRALPAK^{®} AD-H 5 µm
   - Mobile Phase: 80/20 CO₂ / Methanol
   - Flow rate: 60 mL/min
   - Detection: UV 230 nm
   - Outlet Pressure: 150 bar
   - Temperature: 25 °C
   First eluting enantiomer, with a retention time of 5.56 min, was obtained with an enantiomeric excess higher than 99 %, and was identified as *(*4*S*, 5*S)*-5-(4-chlorophenyl)-1-(2,4-dichlorophenyl)-4-methyl-4,5-dihydro-1H-pyrazole-3-carboxylic acid (6.70 g, 47.8 % yield, [α]_{D}= +130.9 °). Second eluting enantiomer, with a retention time of 8.51 min, was also obtained with an enantiomeric excess higher than 99 %, and was identified as *(*4*R*, 5*R)*-5-(4-chlorophenyl)-1-(2,4-dichlorophenyl)-4-methyl-4,5-dihydro-1H-pyrazole-3-carboxylic acid (6.55 g, 46.8 % yield, [α]_{D}= -128.5 °).
b) Diastereomeric resolution with chiral amines 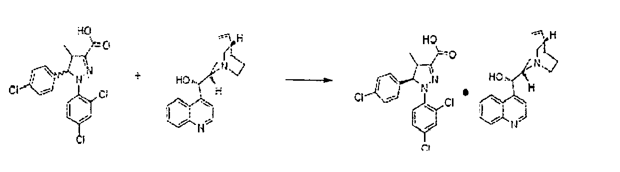 To a suspension of (+)-cinchonine in refluxing MeCN (2 mL), a solution of the racemate *cis-*5-(4-chlorophenyl)-1-(2,4-dichlorophenyl)-4-methyl-4,5-dihydro-1H-pyrazole-3-carboxylic acid (2.0 g, 5.2 mmol) in MeCN (2 ml) was added. To this mixture MeCN was added in small portions until all solids were dissolved. The solution was allowed to cool to r.t. overnight. White crystals were formed, collected by filtration and washed with MeCN. The crystals were dried under vacuum to obtain mainly *(*4*R*, 5*R)*-5-(4-chlorophenyl)-1-(2,4-dichlorophenyl)-4-methyl-4,5-dihydro-1H-pyrazole-3-carboxylic acid (1.7 g, 2.51 mmol, 48% (50% max), ee= 88.3 %). The acid was liberated by extraction of the white solid in toluene and 6 N HCl. The organic layer was dried over Na₂SO₄ and concentrated to give a light yellow foam (0.95 g, 2.47 mmol, 48 % yield, ee= 91.7%). To a solution of this foam in refluxing MeCN (2 ml), (+)-cinchonine (765 mg, 2.60 mmol) was added in a second crystallization process. To this mixture MeCN was added in small portions until all solids were dissolved. The solution was allowed to cool to r.t. overnight. White crystals were formed, collected by filtration and washed with MeCN. The crystals were dried under vacuum. The acid was liberated by extraction in toluene and 6 N HCl to give a white solid identified as pure *(*4*R*, 5*R)*-5-(4-chlorophenyl)-1-(2,4-dichlorophenyl)-4-methyl-4,5-dihydro-1H-pyrazole-3-carboxylic acid (860 mg, 42% yield, ee= 99.5%, [α]_{D}= -128.5 °). Recrystallization of mother liquors led to the (4*S*, 5*S*) enantiomer.

### Chemical-Example 4: Preparation of (4S,5S)-5-(4-chlorophenyl)-1-(2,4-dichlorophenyl)-N-(cis-2,6-dimethylpiperidin-1-yl)-4-methyl-4,5-dihydro-1H-pyrazole-3-carboxamide

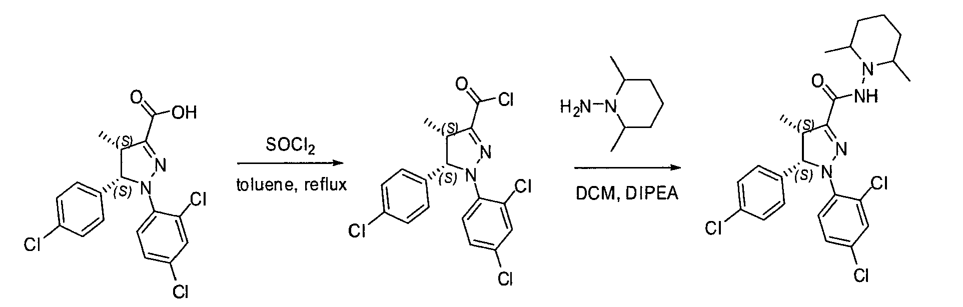

(4*S*,5*S*)-5-(4-chlorophenyl)-1-(2,4-dichlorophenyl)-4-methyl-4,5-dihydro-1H-pyrazole-3-carboxylic acid (20.0 g, 52.13 mmol) obtained according to the step above was dissolved in dry toluene (120 mL) and thionyl chloride (4.5 mL, 62.56 mmols) was added. The mixture was heated to 80 °C for 2.5 hours. The solvent was removed under reduced pressure and the resulting crude residue was used without any further purification.

(4*S*,5*S*)-5-(4-chlorophenyl)-1-(2,4-dichlorophenyl)-4-methyl-4,5-dihydro-1H-pyrazole-3-carbonyl chloride was obtained as an oil.

IR (NaCl film, cm⁻¹) : 1732, 1700, 1533, 1478, 1212, 826.

Under nitrogen atmosphere *cis*-2,6-dimethylaminopiperidine (8.01 g, 62.56 mmoles) and diisopropylethylamine (DIPEA) (2.695 g, 208.5 mmol, 35.7 mL) were dissolved in methylene chloride (DCM) (100 mL). The resulting mixture was ice-cooled down to 0°C and a solution of (4*S*,5*S*)-5-(4-chlorophenyl)-1-(2,4-dichlorophenyl)-4-methyl-4,5-dihydro-1H-pyrazole-3-carbonyl chloride (20.96 g, 52.13 mmol), obtained in the former step, in methylene chloride (50 mL) was added dropwise. The resulting reaction mixture was stirred at room temperature (approximately 25 °C) overnight. Afterwards, the reaction mixture was washed with water, followed by a saturated aqueous solution of sodium bicarbonate, then again with water, dried over sodium sulfate, filtered and evaporated to dryness in a rotavapor. The resulting crude was purified by silicagel chromatography using a CombiFlash Companion apparatus, using a gradient of cyclohexane and ethyl acetate to obtain a white solid (18.3 g, 71 % yield).

1 H NMR (300 MHz, DMSO-d6) δ ppm 0.73 (d, J=7.18 Hz, 3 H) 0.95 (d, J=6.01 Hz, 6 H) 1.23 (br. s., 3 H) 1.63 (m, 3 H) 2.55 (br. s., 2 H) 3.83 (dd, J=11.13, 7.18 Hz, 1 H) 5.88 (d, J=11.13 Hz, 1 H) 7.12 (d, J=8.35 Hz, 2 H) 7.30 (m, 3 H) 7.45 (d, J=2.20 Hz, 1 H) 7.67 (d, J=8.79 Hz, 1 H) 8.64 (br. s., 1 H). MS (M+H)+: 493. [α]D= +57.19 °.

Alternatively, a solution of 2 N HCl in diethyl ether were added over the solid dissolved in diethyl ether to form the hydrochloride, which was collected by filtration.

1 H NMR (300 MHz, DMSO-d6) δ ppm 0.75 (d, J=7.32 Hz, 3 H) 1.05 (d, J=6.01 Hz, 6 H) 1.33 (m, 1 H) 1.47 (m, 2 H) 1.61 (d, J=9.96 Hz, 1 H) 1.72 (m, 2 H) 2.96 (br. s., 2 H) 3.87 (m, 1 H)) 5.92 (d, J=11.28 Hz, 1 H) 7.14 (d, J=8.35 Hz, 2 H) 7.32 (m, 3 H) 7.48 (d, J=2.20 Hz, 1 H) 7.65 (d, J=8.64 Hz, 1 H) 9.80 (br. s., 1 H). MS (M+H)+: 493.

### Chemical-Example 5: Preparation of (4S,5S)-5-(4-chlorophenyl)-1-(2,4-dichlorophenyl)-N-(trans-2,6-dimethylpiperidin-1-yl)-4-methyl-4,5-dihydro-1H-pyrazole-3-carboxamide

The preparation of (4*S*,5*S*)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide was done in an analoguous way to the preparation of (4*S*,5*S*)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide according to Chemical-Example 4.

## Claims

1. Solid dispersion and/or solid solution of one or more active principles selected from compounds of formula (I), (Ia) or (Ib) or mixtures thereof 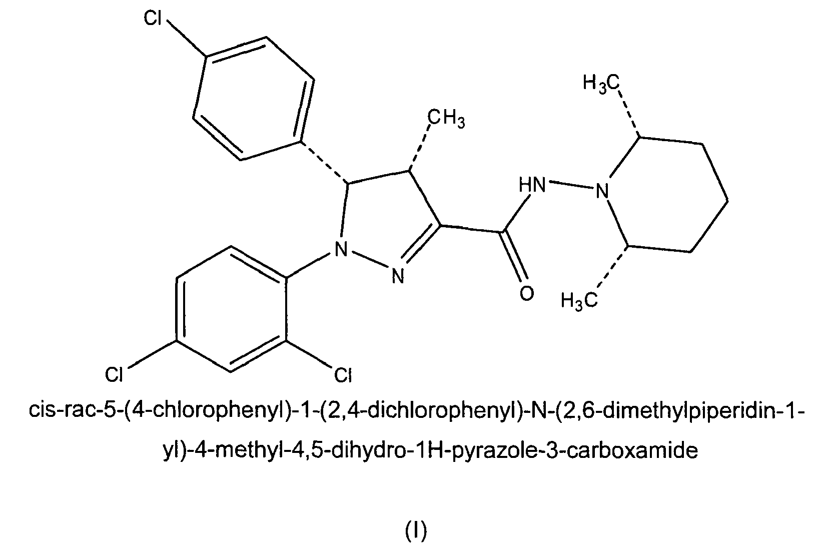 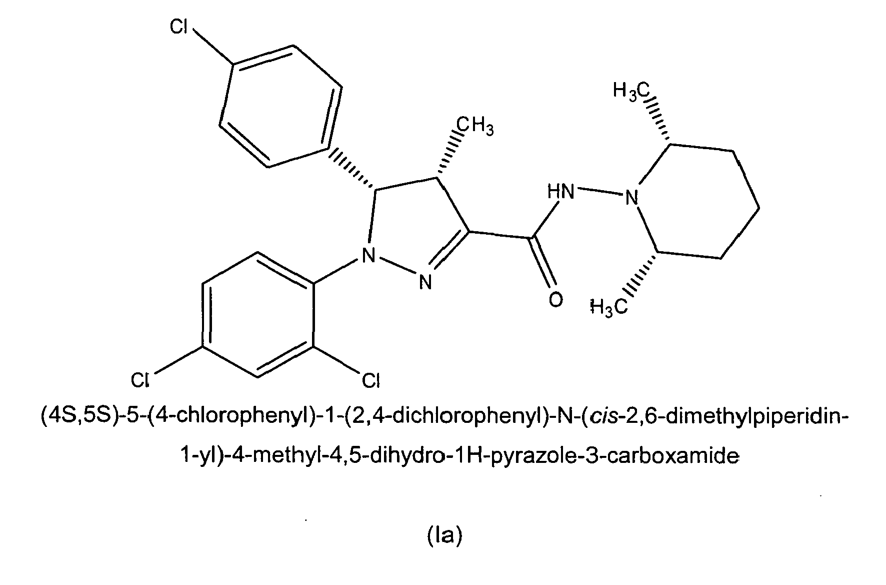 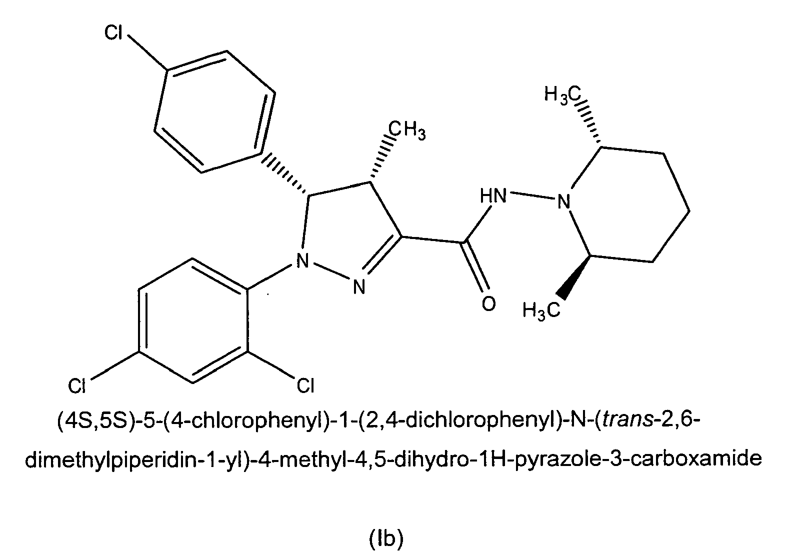 , optionally in form of one or more of the appropriate polymorphs, in amorphous form and/or corresponding salts and/or corresponding solvates thereof,
obtainable by a process in which
a) the active principle/s is/are mixed with an at least equimolar amount of at least one carrier,
b) thereafter or during step a) the mixture is heated until the carrier in the mixture is melted and
c) subsequently the mixture is cooled below the melting point of the carrier in the mixture.

2. Solid dispersion and/or solid solution according to claim 1, **characterized in that** the carrier is either hydrophilic or hygroscopic and has a melting point between +40°C and the melting point of the active principle or mixture of active principles +20 °C.

3. Solid dispersion and/or solid solution according to claim 1, **characterized in that** the carrier is selected from
• sugars, like dextrose, sucrose, galactose, sorbitol, maltose, xylitol, mamitol, lactose;
• acids, like citric acid, succinic acid;
• polymeric materials, like povidone (PVP), polyethylene oxide, polyethylene glycol (PEG), hydroxpropylmethylcellulose, methylcellulose, ethylcellulose hydroxyethylcelllose, cylodextrin, hydroxypropylcellulose, pectin, galactomannan;
• insoluble or enteric polymers, like hydroxypropylmethylcellulose phthalate, polymethacrylates (e.g. Eudragit L-1 00, Eudragit S-1 00, Eudragit RL, Eudragit RS, Eudragit EPO);
• surfactants, like polyoxyethylene stearate, renex, poloxamer 188, texafor, AIP, deoxycholic acid, polyoxy-ethylene-sorbitan higher fatty acid esters (e.g. Tween, like Tween 80), spans;
• others, consisting of: carnuba wax, pentaerythritol, pentaerythrityltetraacetate, urea, urethane, hydroxyalkylxanthins;
preferably the carriers are selected from
EUDRAGIT, MYRJ 52, VITE-TPGS, GELUCIRE 50/13, HPMC-PHTALATE, HPMC, HEC, HPC-SL, PEO and/or POLOXAMER.

4. Solid dispersion and/or solid solution according to any of claims 1 to 3, **characterized in that**
• the melting temperature in step (b) is in the range between +40°C and the melting point of the active principle or mixture of active principles +20 °C; and/or
• the cooling step (c) is done by lowering the temperature by more than 10°C/sec, preferably 20°C/sec, down to temperatures below 25 °C, preferably below 0 °C; and/or is done by contacting the melt of step (b) with an environment having a temperature of 25°C or lower, preferably 0°C or lower.

5. Solid dispersion and/or solid solution according to claim 1, **characterized in that** the molecular ratio between carrier and active principle is between 1:1 and 1: 20, preferably is between 1:1 and 1:10, more preferably is between 1:2 and 1:5.

6. A pharmaceutical composition comprising the solid solution and/or solid dispersion according to any of claims 1 to 5 and optionally further pharmaceutical acceptable ingredients.

7. A pharmaceutical composition according to claim 6, **characterized in that**
• the active principle is present in an amount of 10 to 60, preferably 20 to 40 % by weight based on the total weight of the composition;
and/or
• the active principle is present in an amount of 1 to 250 mg, preferably 10 to 200 mg, more preferably 15 to 150 mg in the composition.

8. A pharmaceutical composition comprising the solid solution according to any of claims 6 or 7, **characterized in that** it is suitable to enhance the absorption of the active principle through a mucosa or a physiological membrane.

9. A pharmaceutical composition according to claim 8, **characterized in that** the mucosa is selected from the
• nasal or olfactory mucosa,
• buccal or oral mucosa,
• gastric mucosa,
• intestinal mucosa
• vaginal mucosa, or
• rectal mucosa,
preferably selected from
• gastric mucosa, or
• intestinal mucosa.

10. A pharmaceutical composition according to any of claims 6 to 9, **characterized in that** the pharmaceutical composition is selected from
• a pharmaceutical composition for oral application,
• a pharmaceutical composition for nasal application,
• a pharmaceutical composition for buccal application,
• a pharmaceutical composition for rectal application, or
• a pharmaceutical composition for vaginal application;
or
• a pharmaceutical composition for transdermal application;
• a pharmaceutical composition for systemic application;
preferably selected from
• a pharmaceutical composition for oral application.

11. A pharmaceutical composition according to any of claims 6 to 10, **characterized in that** the pharmaceutical composition is selected from
• a tablet,
• a capsule,
• a sachet,
• a powder,
• a caplet,
• a gel,
• a film,
• a pellet,
• a granule,
• an implant,
• a multiparticulate with granules compressed into a tablet,
• a multiparticulate with granules filled into a capsule,
• a multiparticulate with pelets compressed into a tablet,
• a multiparticulate with pelets filled into a capsule, or
• a suppository,
or
• an injectable solution/dispersion
• a transdermal system like a patch;
preferably selected from
• a tablet,
• a capsule,
• a pellet,
• a granule,
• a multiparticulate with granules compressed into a tablet,
• a multiparticulate with granules filled into a capsule,
• a multiparticulate with pelets compressed into a tablet, or
• a multiparticulate with pelets filled into a capsule.

12. A pharmaceutical composition according to any of claims 6 to 11, **characterized in that**
• the further pharmaceutical acceptable ingredients are present in a total amount of 5 to 95 %, preferably 50 to 90 % by weight based on the total weight of the composition;
and/or
• the further pharmaceutical acceptable ingredients are present in a total amount of 0 to 300 mg, preferably 0 to 250 mg, more preferably 20 to 250 mg.

13. A pharmaceutical composition according to any of claims 6 to 12, **characterized in that** the further pharmaceutical acceptable ingredient/s is/are selected from
a diluent, a binder and/or a lubricant, and/or optionally a flowing agent, an antiadhesive, a preservative, and/or, optionally, a taste masking agent, a coloring agent and/or a flavoring agent and/or a permeation enhancer.

14. A pharmaceutical composition according to any of claims 6 to 13, **characterized in that** the pharmaceutical composition is prepared using hot-melt extrusion.

15. A pharmaceutical composition for oral administration comprising the solid solution and/or solid dispersion according to any of claims 1 to 5 and optionally further pharmaceutical acceptable ingredients.

16. A solid dispersion and/or solid solution according to any of claims 1 to 5 or a pharmaceutical composition according to any of claims 6 to 14 and 15, **characterized in that** the active principle is selected from
a) (rac)- 5-(4-chlorophenyl)-1-(2,4-dichlorophenyl)-N-(2,6-dimethylpiperidin-1-yl)-4-methyl-4,5-dihydro-1H-pyrazole-3-carboxamide;
preferably in
• cystalline form;
• amorphous form;
• form of a solvate, preferably a hydrate, more preferably a monohydrate;
• the free base; or
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
b) (4s,5s)-5-(4-chlorophenyl)-1-(2,4-dichlorophenyl)-N-(*cis*-2,6-dimethylpiperidin-1-yl)-4-methyl-4,5-dihydro-1H-pyrazole-3-carboxamide, preferably
preferably in
• cystalline form;
• amorphous form;
• form of a solvate, preferably a hydrate,
• the free base;
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
c) (4s,5s)-5-(4-chlorophenyl)-1-(2,4-dichlorophenyl)-N-(*trans*-2,6-dimethylpiperidin-1-yl)-4-methyl-4,5-dihydro-1H-pyrazole-3-carboxamide,
preferably in
• crystalline form;
• form of a solvate, preferably a hydrate,
• the free base;
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsuffamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
or
d) a nonracemic mixtures of (b) and (c).
